# EUROPEAN PATENT SPECIFICATION

(11) **EP 2 749 657 B1**
(45) Date of publication and mention of the grant of the patent: **31.10.2018**
(21) Application number: 12382542.4
(22) Date of filing: 27.12.2012
(51) Int. Cl.: G01N 25/04, C21C 1/10, G01N 33/20

(54) **Method for controlling active magnesium in ductile cast iron**
Verfahren zum Steuern von aktivem Magnesium in leitfähigem Gusseisen
Procédé de régulation active du magnésium dans de la fonte ductile

(43) Date of publication of application: 02.07.2014
(73) Proprietor: Veigalan Estudio 2010 S.L.U., 48200 Durango (Vizcaya) (ES); Thermal Quality Control Technologies S.L.U., 48200 Durango (Vizcaya) (ES); Casa Maristas Azterlan, 48200 Durango, Vizcaya (ES)
(72) Inventor: Suárez Creo , Ramón, 48200 Durango (Vizcaya) (ES); Garay Abascal, Jon, 48200 Durango (Vizcaya) (ES); Larrañaga Zarraoa, Pello, 48200 Durango (Vizcaya) (ES)
(74) Representative: Stiebe, Lars Magnus

(56) References cited:
- EP-A2- 0 327 237
- WO-A1-98/25133
- DE-A1- 10 004 910

## Description

### FIELD OF THE INVENTION

The invention relates to ductile iron or spheroidal graphite, and more particularly relates to a method for controlling active magnesium in the melt.

### BACKGROUND OF THE INVENTION

Ductile iron foundry shops are particularly concerned about how to assure that the proper degree of nodularity is achieved for every part produced without sacrificing production rate, and optimizing production time and costs.

Ductile iron is made by treating the liquid cast iron in a ladle or in a special treatment vessel with "nodulazing" elements which promote the precipitation of graphite in the spheroidal shape. Magnesium has proved to be a very efficient element and is the most common one used in ductile iron manufacturing processes.
Magnesium is a highly reactive element, so that it rapidly combines mainly with oxygen and sulfur. Its boiling point is about 1090ºC, much below the usual nodulizing treatment temperature (1380-1480ºC). Those aspects provoke a low yield of the treatment, which depends on the product and methodology used. There are many parameters that affect to this yield: temperature of the treatment, amount of melt treated, chemical composition, etc. Therefore in industrial conditions it is quite difficult to control its amount, considering that after the nodulizing treatment, the loss of magnesium continues during time due to its combination mainly with oxygen and sulfur and its vapourization to the atmosphere.
Only the magnesium that is in a free state (called active Mg) is prone to graphite shape modification. Active Mg thus, has to be in a determined amount in the melt to success in achieving nodular graphite, while when it is in excess, it promotes shrinkage appearance and too many inclusions, that in addition to higher production costs, cause non mechanical properties requirement fulfilling and therefore scrap production. For those reasons, its control is of primarily importance, to ensure that the desired degree of nodularity is achieved, and that there is not too much magnesium that gives rise to some other problems.

One of the methods commonly used in ductile iron foundry shops for assuring the presence of enough active magnesium in the melt to obtain ductile iron castings is the metallographic inspection of a sample poured from the same melt. This methodology is time and resources consuming, and does not allow to fully control all the batches. Another procedure that can be applied is the spark spectrometry, which involves the obtaining of a quenched sample (white iron sample). This last technique gives the total amount of magnesium (residual Mg) which is the sum of the one that is in a free state and the one which is combined. A further method of the state of the art, and which is currently used in some shops, is based on a thermal analysis technique for the purpose of testing the active magnesium content in the melt after treatment (see EP 0 327 237). The method determines if the active magnesium content of the liquid iron is above or below a previously established level.

The method uses specially prepared disposable thermal analysis cups containing a controlled amount of sulfur and a controlled amount of tellurium and are provided with a standard immersion thermocouple. EP 0 327 237 discloses that tests are conducted after magnesium treatment before or after inoculation to check the active magnesium level before pouring the metal in the molds. A cast sample is poured in a thermal analysis cup, and during its solidification the cooling curve is registered. If the alloy solidifies as white iron, this means that there is not active Mg available, and so tellurium promotes a fully carbidic alloy and the system predicts a Mg level below the established one. If the alloy solidifies as graphitic iron (not white), this indicates that there is some active Mg and therefore the tellurium cannot promote a fully carbidic alloy, so the Mg level is above the established level. Those cooling curves (graphitic and carbidic) are easily distinguishable.

This method however suffers from some drawbacks, leading sometimes to inaccurate results, either false-positives or false-negatives. In this sense the inventors have noticed that there are some cases in foundry shops where this test leads to a negative result, although the real amount of active magnesium present in the liquid iron is such, that the test should give a positive result. The opposite case, in which a positive result is obtained although a negative should be obtained, is also possible.

In view of the above there is still a need in the state of the art to provide an alternative method for controlling the amount of active magnesium present in a treated melt and in particular for determining if the amount of active magnesium is above or below a established level, which overcomes at least part of the above mentioned drawbacks. WO98/25133 discloses another method to control the quality of the melt.

### DESCRIPTION OF THE DRAWINGS

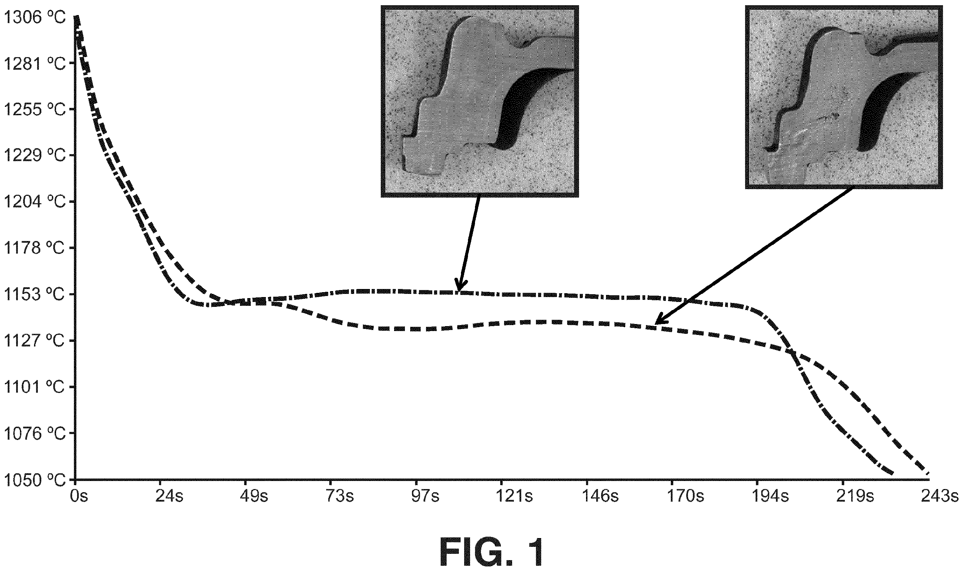

Figure 1 is a graph showing the cooling curves (T^{a} (ºC) vs. time (seconds)) of two thermal analysis of two different test samples with the same chemical composition of the metal treated with the same Mg alloy but of different metallurgical quality. Figure 1 also shows the metallography of the parts manufactured: a) with the metal of good metallurgical quality without defects, and b) with the metal with low metallurgical quality with defects.

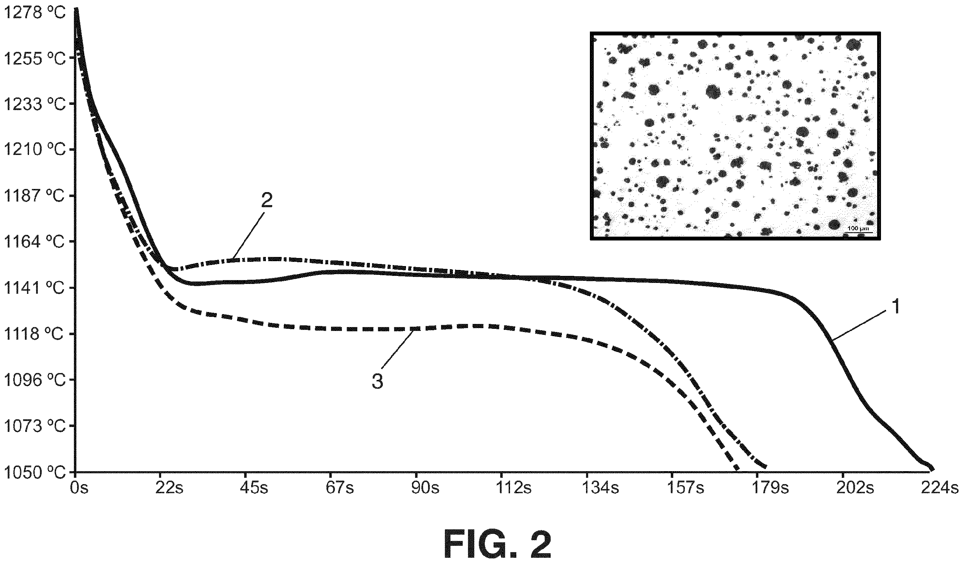

Figure 2 is a graph showing the cooling curves of one positive trial, with three channels, where the amount of active Mg in the melt is enough to produce ductile iron castings, as shown in the metallography of the corresponding casting.

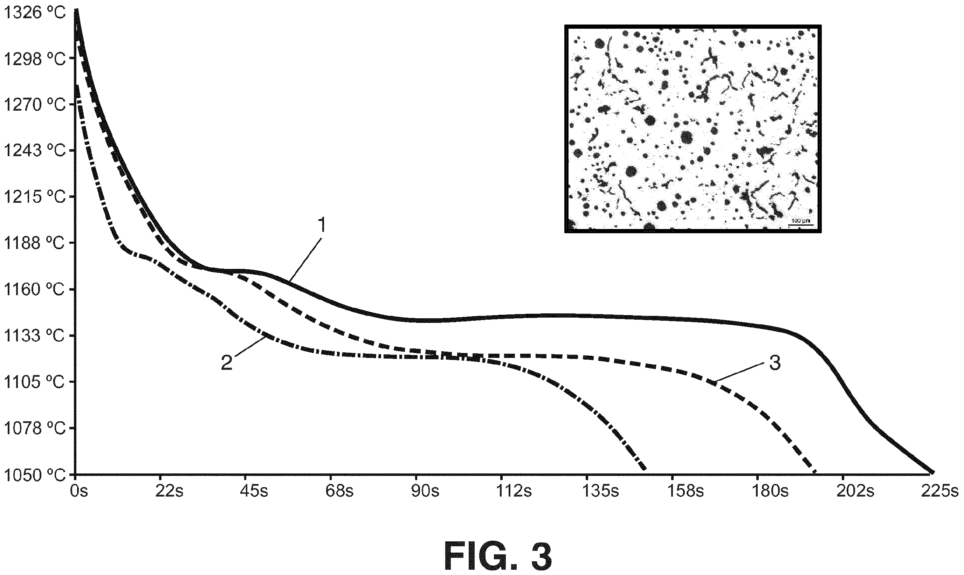

Figure 3 is a graph showing the cooling curves of one negative trial, with three channels, where the amount of active Mg in the melt is insufficient to produce spheroidal graphite part, as shown in the metallography of the corresponding casting.

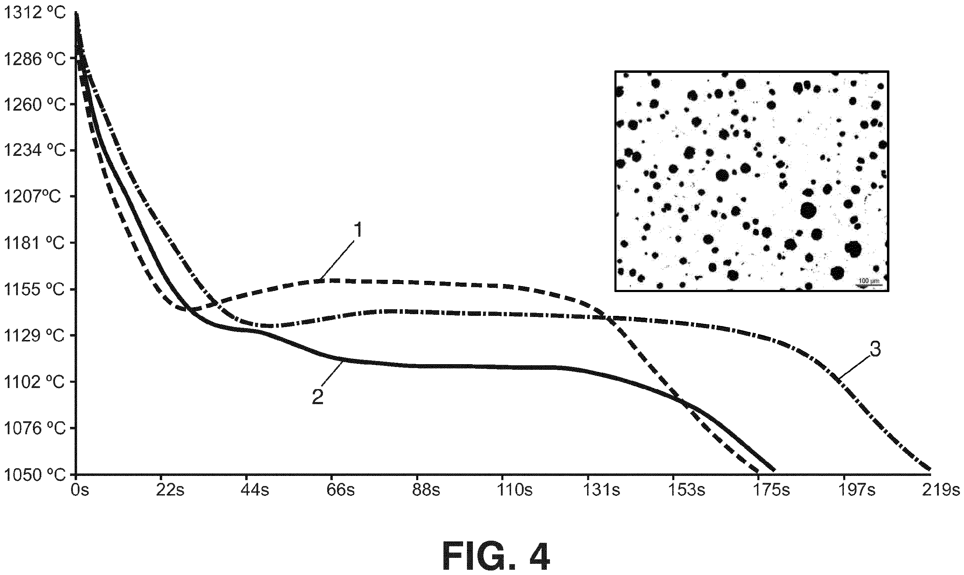

Figure 4 is a graph showing the cooling curves from the comparative Example 2, with three channels, where the amount of active Mg in a melt of low metallurgical quality is sufficient to produce spheroidal graphite part using: a thermal analysis cup only with sulfur, and a thermal analysis cup according to the present invention. The metallographic inspection of the obtained spheroidal graphite part is also shown.

### DESCRIPTION OF THE INVENTION

In a first aspect the present invention relates to a method for controlling the amount of active magnesium present in a treated melt. This method, hereinafter referred to as the method of the invention is able to accurately determine if the active magnesium content of the treated melt is above or below a certain level. Said level is established by the own foundry shop according to their needs for its ductile iron, and assures that the ductile iron castings obtained have the desired nodularity rate. Levels are expressed in the state of the art as percentages of active or uncombined magnesium and are typically between 0.020% to 0.040%. Usually convenient nodularity rates for ductile iron are equal or above 90%.

The method of the invention comprises the following steps:
(i) adding to a thermal analysis cup with tellurium provided with an immersion thermocouple and a metallic cap, a controlled amount of a neutralizing mixture consisting of sulfur and FeSi, said controlled neutralizing mixture comprising:
   a) from 0.07 to 0,25 wt% of FeSi in respect of the weight of the test sample of the treated melt which is poured into it in the next step (ii), and
   b) a number of equivalents of sulfur which is the same as the number of equivalents of active magnesium which correspond to the said certain level to be determined,
(ii) pouring into the thermal analysis cup an amount of treated melt to fill the cup;
(iii) recording a cooling curve from the test sample, and
(iv) identifying according to the shape of the curve, if the test sample has solidified at the graphite eutectic or at the carbide eutectic.

The thermal analysis cups with tellurium to be used in the present method are standard commercially available cups, which can be acquired from different companies, such as Electronite, Heraeus. The amount of tellurium is determined by the necessity that he iron solidifies at the metastable temperature (carbidic eutectic). They comprise 20-55 wt% of tellurium in respect of the weight of the test sample of the treated melt, which is poured into the cup. They have capacity for amounts of melt of 360 g ± 10%. These cups are provided with an immersion thermocouple, such as type K. Tellurium is a metal element with a low melting point, which turns to gas phase when it gets into contact with the melt. The neutralizing mixture consists of sulfur and FeSi. The FeSi used is FeSi40-75%. According to another particular embodiment is FeSi69%.
The neutralizing mixture is prepared as follows: FeSi is milled to obtain a powder which is sieved though a 160 µm sieve, resulting in a homogeneous powder with the same grain size than the sulfur. The needed amounts of sulfur and of this homogenous powder are weight and thoroughly mixed.

The neutralizing mixture is introduced in the cups. The cup contains a controlled amount of a neutralizing mixture having between 14 to 40 wt.% of sulfur, the rest being FeSi. The cup contains a controlled amount of a neutralizing mixture consisting of 22 ±1 wt.% of sulfur and 78 ± 1 wt.% of FeSi.
The recording of a cooling curve from the test sample, is carried out in a conventional way. The recorded shape of the curve indicates if the test sample has solidified according to the stable model (graphitic eutectic) or the metastable model (carbidic eutectic). In the first case the test sample solidifies at the graphite eutectic and the shape of the resulting curve determines that the amount of active magnesium present in the treated melt is above the established level. In the second case, the test sample solidifies at the carbide eutectic. The resulting curve shows that the sample solidifies as white iron, determining that the amount of active magnesium is below the established level.
The method of the present invention can be put in practice using Thermolan® a support tool which is commercially available, which displays on a screen a "OK" or "No OK" message, and which allows an operator to decide quickly on the spot, within a period of time of 2-3 minutes after pouring the test sample, whether to go on with the casting process when the message is "OK", or to increase the magnesium content in the melt to achieve the desired magnesium when the message is "No-OK". The "OK" message can also be taken as an indication that it is possible to reduce the active magnesium level in the melt to reduce costs.
A particular embodiment of the method of the invention is for controlling if the amount of active magnesium in a treated melt is above or below a certain first level and is above or below a certain second level. That is the method is used to determine whether the amount of active magnesium in a treated melt is comprised with certain desired range. Said range is established by each particular foundry shop according to their needs.

According to this particular embodiment the method comprises the following steps:
(i) adding to a first thermal analysis cup with tellurium provided with an immersion thermocouple and a metallic cap, a first controlled amount of a neutralizing mixture of sulfur and FeSi, said first controlled neutralizing mixture comprising:
   a) from 0.07 to 0.25 wt% of FeSi in respect of the weight of the test sample of the treated melt which is poured into it in the next step (ii), and
   b) a number of equivalents of sulfur which is the same as the number of equivalents of active magnesium which correspond to the said first certain level to be determined,
(ii) pouring into the thermal analysis cup an amount of treated melt to fill the cup;
(iii) recording a cooling curve from the test sample,
(iv) identifying according to the shape of the curve, if the test sample has solidified at the graphite eutectic or at the carbide eutectic; and
(v) carrying out the same steps (i) to (iv) stated, with a second thermal analysis cup and with a second controlled amount of a neutralizing mixture, which comprises a number of equivalents of sulfur which is the same as the number of equivalents of active magnesium which correspond to the said second certain level to be determined.

The method of the present invention shows an important advantage, as it takes into account the possible different metallurgical qualities of the melt to be tested.

The metallurgical quality is a very important aspect in the manufacturing of ductile iron castings. Several factors depend on the metallurgical quality of the alloy such as the nucleating power, the graphitization capacity, the tendency to carbides formation and the tendency to a secondary contraction of the metal. All of these characteristics have a significant influence on the development of defects in the castings and performance of active magnesium control tests. Regarding the latter if the nucleation ability of the melt is too poor, graphite cannot precipitate, and a fully carbidic iron (white iron) is obtained independently of the magnesium level. This drawback is overcome by the FeSi addition.

The metallurgical quality of a metal fluctuates in a foundry shop during the fabrication process and it depends on many factors, among which, temperature and time can be mentioned.

This can be seen in a test shown in Figure 1. The sampling of metal is carried out in the pouring area. The chemical composition of the metal treated with Mg alloy is the same for the two channels. The castings manufactured with the metal of one of the channels are correct, without defects, while defects are observed in the parts manufactured with the metal of the other channel. The sections of the castings associated to each channel are shown in figure 1. These results prove the influence of the metallurgical quality in the active magnesium determination.

The present invention takes this fact into account, and the impact of the mentioned factors, which when not taken into account may lead sometimes to false-positives or false-negatives as explained above.

Accordingly, the method of the present invention shows the advantage that it can be carried out at any time during the manufacturing process after magnesium treatment, and its positive or negative result is not affected by events which may occur in a foundry shop such as stops or delays in production, fading of magnesium, poor melt quality, etc. According to a particular embodiment the method is carried out before inoculation. According to another particular embodiment the method is carried out after inoculation.

In this way decisions may be taken by the operator early enough in the manufacturing process to act according to the test result, before pouring the castings.

The method is cost effective, rapid, and increases the quality of casting, yield, and production rate.

The following examples are non-limiting and are merely representative of various aspects of the invention.

### EXAMPLES

### Example 1

The results of two control spheroidization trials with the same metal treated with Mg ferroalloy are shown in Figures 2 and 3.
Three tests have been carried out in each trial, and the cooling curves registered:
(i) channel 1 with thermal analysis cup (Heraus Electronite) just to register the cooling of the melt;
(ii) channel 2 a thermal analysis cup prepared for predicting the active magnesium (with S+FeSi) for 0,023% of active magnesium.
(iii) channel 3 with a thermal analysis cup that contains Te and S in excess for neutralizing 0,023% of active magnesium to predict the chemical composition (%C, %S).
The channel 3 controls a defined active magnesium necessary to manufacture spheroidal graphite parts.

Trial 1 was positive (Figure 2) while the trial 2 (Figure 3) was negative. This means, that the amount of active Mg in the liquid metal of the trial 1 was enough to manufacture a spheroidal graphite part, as shown in the micrograph of the part, however the amount of the active magnesium in the trial 2 was insufficient as shown in the micrograph of the part.

### Example 2

Comparative example: Method for controlling if the amount of uncombined (active) magnesium in a treated melt is above or below a certain level (0,023%) enough to render a spheroidal graphite casting for a melt with a low metallurgical quality (results are shown in Figure 4). The following tests were carried out in a foundry plant.
Three equal samples of the melt were collected in the pouring area after magnesium treatment.
The channel 1 corresponds to a thermal analysis cup with Te and the neutralizing mixture (S + FeSi) for an active magnesium control 0,023%. The channel 2 corresponds to a thermal analysis cup with Te and S for neutralizing the same magnesium amount (0,023%) according to the state of the art. The channel 3 is a thermal analysis cup without elements inside and shows the solidification curve of the treated metal. The metallurgical quality can be seen to be low.

The channel 1 was positive; this means that the magnesium amount was enough to manufacture spheroidal parts. The channel 2 was negative, meaning that a manufactured casting was not a ductile iron casting, indicating that there was not enough magnesium to manufacture spheroidal parts.

However, as shown in Figure 4, the metallographic characterization of the manufactured castings show that spheroidal graphite particles were obtained in both cases, (see micrograph in Figure 4) demonstrating that the method of the state of the art can lead to false-negatives. It can be seen that although the amount of active magnesium in the melt was in fact enough to render spheroidal graphite particles, the test results obtained were different depending on the metallurgical quality.

## Claims

1. A method for controlling if the amount of active magnesium in a treated melt of ductile iron or spheroidal graphite is above or below a certain level which comprises the following steps:
(i) adding to a thermal analysis cup with tellurium provided with an immersion thermocouple and a metallic cap, a controlled amount of a neutralizing mixture of sulfur and FeSi, said controlled neutralizing mixture comprising:
a) from 0.07 to 0.25 wt% of FeSi in respect of the weight of the test sample of the treated melt which is poured into it in the next step (ii), and
b) a number of equivalents of sulfur which is the same as the number of equivalents of active magnesium which correspond to the said certain level to be determined,
(ii) pouring into the thermal analysis cup an amount of treated melt to fill the cup;
(iii) recording a cooling curve from the test sample, and
(iv) identifying according to the shape of the curve, if the test sample has solidified at the graphite eutectic or at the carbide eutectic.

2. A method according to claim 1 for controlling if the amount of active magnesium in a treated melt is above or below a certain first level and above or below a certain second level which comprises the following steps:
(i) adding to a first thermal analysis cup with tellurium provided with an immersion thermocouple and a metallic cap, a first controlled amount of a neutralizing mixture of sulfur and FeSi, said first controlled neutralizing mixture comprising:
a) from 0.07 to 0.25 wt% of FeSi in respect of the weight of the test sample of the treated melt which is poured into it in the next step (ii), and
b) a number of equivalents of sulfur which is the same as the number of equivalents of active magnesium which correspond to the said first certain level to be determined,
(ii) pouring into the thermal analysis cup an amount of treated melt to fill the cup;
(iii) recording a cooling curve from the test sample,
(iv) identifying according to the shape of the curve, if the test sample has solidified at the graphite eutectic or at the carbide eutectic; and
(v) carrying out the same steps (i) to (iv) with a second thermal analysis cup with a second controlled amount of a neutralizing mixture, which comprises a number of equivalents of sulfur which is the same as the number of equivalents of active magnesium which correspond to the said second certain level to be determined.

3. A method according to claim 1 or 2, wherein the controlled neutralizing mixture has 14-40 wt.% of sulfur, the rest being FeSi.

4. A method according to claim 3, wherein the controlled neutralizing mixture consists of 22 ±1 wt.% of sulfur and 78 ± 1 wt.% of FeSi.

## Patentansprüche

1. Verfahren zum Kontrollieren, ob die Menge von aktivem Magnesium in einer behandelten Schmelze aus duktilem Eisen oder Kugelgraphit oberhalb oder unterhalb eines gewissen Niveaus liegt, das die folgenden Schritte umfasst:
(i) Hinzufügen einer kontrollierten Menge einer neutralisierenden Mischung aus Schwefel und FeSi zu einem Thermoanalysebecher mit Tellur, der mit einem Tauchthermoelement und einer metallischen Kappe versehen ist, wobei die kontrollierte neutralisierende Mischung umfasst:
a) von 0,07 bis 0,25 Gew.-% von FeSi in Bezug auf das Gewicht der Untersuchungsprobe der behandelten Schmelze, die im nächsten Schritt (ii) in ihn hineingegossen wird, und
b) eine Anzahl von Äquivalenten von Schwefel, die dieselbe ist wie die Anzahl von Äquivalenten von aktivem Magnesium, die dem besagten gewissen Niveau entspricht, das zu bestimmen ist,
(ii) Gießen einer Menge der behandelten Schmelze in den Thermoanalysebecher, um den Becher zu füllen;
(iii) Aufzeichnen einer Abkühlkurve von der Untersuchungsprobe; und
(iv) gemäß der Form der Kurve Feststellen, ob sich die Untersuchungsprobe beim Graphit-Eutektikum oder beim Carbid-Eutektikum verfestigt hat.

2. Verfahren nach Anspruch 1 zum Kontrollieren, ob die Menge von aktivem Magnesium in einer behandelten Schmelze oberhalb oder unterhalb eines gewissen ersten Niveaus und oberhalb oder unterhalb eines gewissen zweiten Niveaus liegt, das die folgenden Schritte umfasst:
(i) Hinzufügen einer ersten kontrollierten Menge einer neutralisierenden Mischung aus Schwefel und FeSi zu einem ersten Thermoanalysebecher mit Tellur, der mit einem Tauchthermoelement und einer metallischen Kappe versehen ist, wobei die erste kontrollierte neutralisierende Mischung umfasst:
a) von 0,07 bis 0,25 Gew.-% von FeSi in Bezug auf das Gewicht der Untersuchungsprobe der behandelten Schmelze, die im nächsten Schritt (ii) in ihn hineingegossen wird, und
b) eine Anzahl von Äquivalenten von Schwefel, die dieselbe ist wie die Anzahl von Äquivalenten von aktivem Magnesium, die dem besagten ersten gewissen Niveau entspricht, das zu bestimmen ist,
(ii) Gießen einer Menge der behandelten Schmelze in den Thermoanalysebecher, um den Becher zu füllen;
(iii) Aufzeichnen einer Abkühlkurve von der Untersuchungsprobe,
(iv) gemäß der Form der Kurve Feststellen, ob sich die Untersuchungsprobe beim Graphit-Eutektikum oder beim Carbid-Eutektikum verfestigt hat; und
(v) Ausführen derselben Schritte (i) bis (iv) mit einem zweiten Thermoanalysebecher mit einer zweiten kontrollierten Menge einer neutralisierenden Mischung, die eine Anzahl von Äquivalenten von Schwefel umfasst, die dieselbe ist wie die Anzahl von Äquivalenten von aktivem Magnesium, die dem besagten zweiten gewissen Niveau entspricht, das zu bestimmen ist.

3. Verfahren nach Anspruch 1 oder 2, wobei die kontrollierte neutralisierende Mischung 14-40 Gew.-% Schwefel aufweist, wobei der Rest FeSi ist.

4. Verfahren nach Anspruch 3, wobei die kontrollierte neutralisierende Mischung aus 22 ± 1 Gew.-% Schwefel und 78 ± 1 Gew.-% FeSi besteht.

## Revendications

1. Procédé pour contrôler si la quantité de magnésium actif dans une masse fondue traitée de fonte ductile ou de graphite sphéroïdal est supérieure ou inférieure à un certain niveau, procédé qui comprend les étapes suivantes consistant à :
(i) ajouter dans un godet d'analyse thermique avec du tellure muni d'un thermocouple à immersion et d'un couvercle métallique, une quantité contrôlée d'un mélange neutralisant de soufre et de FeSi, ledit mélange neutralisant contrôlé comprenant :
a) de 0,07 à 0,25 % en poids de FeSi par rapport au poids de l'échantillon d'essai de la masse fondue traitée qui y est versé à l'étape suivante (ii), et
b) un nombre d'équivalents de soufre égal au nombre d'équivalents de magnésium actif correspondant audit certain niveau à déterminer,
(ii) verser dans le godet d'analyse thermique une quantité de masse fondue traitée pour remplir le godet ;
iii) enregistrer une courbe de refroidissement de l'échantillon d'essai, et
iv) identifier en fonction de la forme de la courbe, si l'échantillon d'essai s'est solidifié à l'eutectique graphite ou à l'eutectique carbure.

2. Procédé selon la revendication 1 pour contrôler si la quantité de magnésium actif dans une masse fondue traitée est supérieure ou inférieure à un certain premier niveau, et supérieure ou inférieure à un certain second niveau, procédé qui comprend les étapes suivantes consistant à :
(i) ajouter dans un premier godet d'analyse thermique avec du tellure muni d'un thermocouple à immersion et d'un couvercle métallique, une première quantité contrôlée d'un mélange neutralisant de soufre et de FeSi, ledit premier mélange neutralisant contrôlé comprenant :
a) de 0,07 à 0,25 % en poids de FeSi par rapport au poids de l'échantillon d'essai de la masse fondue traitée qui y est versé à l'étape suivante (ii), et
b) un nombre d'équivalents de soufre égal au nombre d'équivalents de magnésium actif correspondant audit certain premier niveau à déterminer,
(ii) verser dans le godet d'analyse thermique une quantité de masse fondue traitée pour remplir le godet ;
iii) enregistrer une courbe de refroidissement de l'échantillon d'essai,
iv) identifier en fonction de la forme de la courbe, si l'échantillon d'essai s'est solidifié à l'eutectique graphite ou à l'eutectique carbure ; et
(v) réaliser les mêmes étapes (i) à (iv) avec un second godet d'analyse thermique avec une seconde quantité contrôlée d'un mélange neutralisant, qui comprend un nombre d'équivalents de soufre égal au nombre d'équivalents de magnésium actif correspondant audit second certain niveau à déterminer.

3. Procédé selon la revendication 1 ou 2, dans lequel le mélange neutralisant contrôlé contient 14 à 40 % en poids de soufre, le reste étant du FeSi.

4. Procédé selon la revendication 3, dans lequel le mélange neutralisant contrôlé est constitué de 22 ± 1 % en poids de soufre et 78 ± 1 % en poids de FeSi.
